# EUROPEAN PATENT SPECIFICATION

(11) **EP 1 755 527 B1**
(45) Date of publication and mention of the grant of the patent: **22.06.2016**
(21) Application number: 05747831.5
(22) Date of filing: 26.04.2005
(51) Int. Cl.: A61J 15/00, A61B 17/34

(54) **GASTROSTOMY TUBE EXTENSION DEVICE**
VORRICHTUNG ZUM AUSZIEHEN EINES GASTROSTOMIE-TUBUS
DISPOSITIF D'EXTENSION DE SONDE DE GASTROSTOMIE

(30) Priority: 30.04.2004 JP 2004135258
(43) Date of publication of application: 28.02.2007
(73) Proprietor: Covidien AG, 8212 Neuhausen am Rheinfall (CH)
(72) Inventor: NAGATA, Katsuki, Nippon Sherwood Medical Ind. Ltd., Fukuroi-shi, Shizuoka 437-0004 (JP); BABA, Yasuyuki, Nippon Sherwood Medical Ind. Ltd., Fukuroi-shi, Shizuoka 437-0004 (JP)
(74) Representative: Tomlinson, Edward James
(86) International application number: PCT/EP2005/004464
(87) International publication number: WO 2005/105017

(56) References cited:
- WO-A-97/34552
- DE-U1- 20 011 230
- US-A- 4 861 334
- US-A- 5 855 569
- US-A1- 2002 165 553

## Description

### [Technical Field]

The present invention relates to a gastrostomy tube extension device used for inserting or taking out a gastrostomy tube into/from a patient's body for feeding fluid substances such as liquid food into a stomach of a patient.

### [Background Art]

Hitherto, feeding of fluid substances such as liquid food or nutrient preparation using a gastrostomy tube has been carried out for persons whose capability to take food orally by their own abilities is deteriorated due to aging or diseases (hereinafter referred to as a "patient"). The gastrostomy tube includes a tube member to be installed at a hole (fistula hole) for dietary intake provided in a patient's body, an inner fixing member, often a catheter balloon, fixed to a distal portion of the tube member and installed inside of a stomach wall and an outer fixing member mounted to a proximal portion of the tube member and installed on the side of the body skin. When inserting or taking out the gastrostomy tube into/from the hole formed on the patient's body, operation for insertion and taking-out thereof is performed by a gastrostomy tube extension device, as disclosed in JP-T-2000-507134. Document WO 9734552 also discloses a gastrointestinal tube insertion or removal device.

The gastrostomy tube extension device includes a pushing rod which can be inserted into and taken out from a fluid substance feed hole formed on the gastrostomy tube, and an engaging member including an outer fixing member engaging portion which can engage the outer fixing member of the gastrostomy tube and a finger hook on which a finger can be hooked. The inner fixing member is formed at the center of the distal end thereof with a pushing rod engaging portion with which the distal portion of the pushing rod can engage, so that the inner fixing member is elongated when the pushing rod is inserted into the fluid substance feed hole and the pushing rod engaging portion is pushed toward the distal end.

Therefore, when the pushing rod is pushed into the fluid substance feed hole while pulling the finger hook of the engaging member toward the outside (toward the proximal side of the gastrostomy tube) with fingers while the outer fixing member engaging portion of the engaging member is engaged with the outer fixing member, the inner fixing member can be elongated and narrowed. Then, since the inner fixing member in this narrowed state can pass through the hole formed on a patient's abdominal part, the gastrostomy tube can be placed in or taken out from the hole. Also, when the inner fixing member is inserted into the interior of the stomach wall and the force from the pushing rod is released by pulling out the pushing rod, the inner fixing member is brought into a swelled state by its resiliency, and hence the gastrostomy tube does not come out from the patient's body.

When inserting or taking out the aforementioned gastrostomy tube into/from a hole formed on the patient's body, it is necessary to manually maintain the position of the engaging member with respect to the pushing rod constant so that the thickness of the inner fixing member can be kept constant. In other words, with the aforementioned gastrostomy tube extension device, the operation to insert or remove the gastrostomy tube requires great skill by the user, who has to maintain the position by hand. In recent years, it has become more common for "patients" to play a greater role in their personal lives; it is now common for those requiring gastrostomy tubes for themselves or their small children to choose to maintain, and even replace, these tubes without the help of a medical doctor or nurse. This greater role of the patients, however, does require that the insertion/removal of the gastrostomy tubes be relatively uncomplicated to complete.

### [Disclosure of the Invention]

In order to solve the above-described problems, it is an object of the present invention to provide a gastrostomy tube extension device which can easily be inserted or taken out. This is achieved by fixing the degree of extension of the gastrostomy tube during the procedure.

Accordingly, the present invention provides a gastrostomy tube extension device according to claim 1. In order to achieve the above-described object, a structural characteristic of the gastrostomy tube extension device according to the present invention is the gastrostomy tube extension device used for inserting or taking out the gastrostomy tube into/from a hole on a patient formed between a skin surface and the inner surface of a stomach wall. The gastrostomy tube includes: an outer fixing member to be installed at the hole on the skin surface side; an inner fixing member to be installed on the inner side of the stomach wall, a tube member for connecting the outer fixing member and a proximal portion of the inner fixing member, and a fluid substance feed hole extending from the outer fixing portion to the inner fixing portion.

A pushing rod formed of a rod member which can be inserted into the fluid substance feed hole, is capable of pushing the center portion of the distal end of the inner fixing member toward the distal end with the distal end portion of the rod, and is formed with a positioning engaging portion on the peripheral surface of the proximal portion.

An engaging member capable of engaging the outer fixing member and the pushing rod, is used to elongate the inner fixing portion when the pushing rod is inserted into the fluid substance feed hole and the outer fixing member, when the pushing rod is in a locked position with respect to the engaging member and the outer fixing member.

In the gastrostomy tube extension device according to the present invention configured as described above, the pushing rod is provided with a positioning engaging portion on the peripheral surface of its proximal portion, which is capable of engaging the engaging member. Therefore, when the pushing rod is inserted into the fluid feed hole of the gastrostomy tube and the outer fixing member of the gastrostomy tube and locked with the engaging member (achieved by pulling the engaging member in the proximal direction of the pushing rod), the inner fixing member is maintained in the narrowed, extended state, due to the pressure the distal end of the pushing rod exerts on the distal end of the inner fixing member. Therefore, the operator who performs the insertion or removal operation of the gastrostomy tube can concentrate on the insertion or removal operation, possibly using only one hand, without paying attention to the degree of extension of the gastrostomy tube. Consequently, insertion and removal of the gastrostomy tube into/from the hole on the patient are facilitated.

Another structural characteristic of the gastrostomy tube extension device according to the present invention is that the inner fixing member to be installed on the inner side of the stomach wall is formed of a resilient material which is elongated when the center of the distal end is pushed toward the distal direction. The distal end of the pushing rod is capable of pushing the center portion of the distal end of the inner fixing member toward the distal end, and is formed with a positioning engaging portion on the peripheral surface of the proximal portion. A further structural characteristic is an engaging portion being capable of engaging the positioning engaging portion of the pushing rod, and elongating and narrowing the inner fixing member when the pushing rod is inserted into the fluid substance feed hole and the positioning engaging portion and the engaging portion are engaged respectively.

The engaging member may include a joint member being connected to the gastrostomy tube. In this case, since the engaging member is connected to the gastrostomy tube via the joint member, the operation to connect the engaging member with the gastrostomy tube is no longer necessary, and the operation of the device is made easier.

Another structural characteristic of the gastrostomy tube extension device according to the present invention is in that a plurality of the positioning engaging portions are provided along the axial direction of the pushing rod. With a plurality of positioning engaging portions, the extended state of the gastrostomy tube can be changed arbitrarily by selecting the positioning engaging portion of the pushing rod to engage the second engaging portion of the engaging member as needed. Accordingly, versatility is provided, and hence one type of gastrostomy tube extension device is suitable for various body types.

Furthermore, adaptation of expansion properties or other physical properties of the gastrostomy tube can be made. For example, when inserting the gastrostomy tube into the hole on the patient, the operation is performed with the second engaging portion engaged with the positioning engaging portion which provides the smallest extension. Then, when taking out the gastrostomy tube from the hole on the patient after having placed the gastrostomy tube in the patient's body, the second engaging portion can be engaged with another positioning engaging portion. Accordingly, the operation of the gastrostomy tube can be performed with the tube in the extension state optimal for each operation.

It is further noted that the insertion/removal procedure with the present invention may be carried out using only one hand, greatly increasing the freedom of patients maintaining their gastrostomy tubes themselves.

### [Best Mode for Carrying Out the Invention]

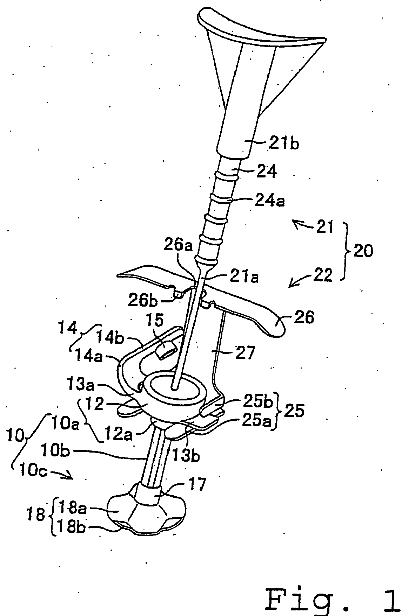

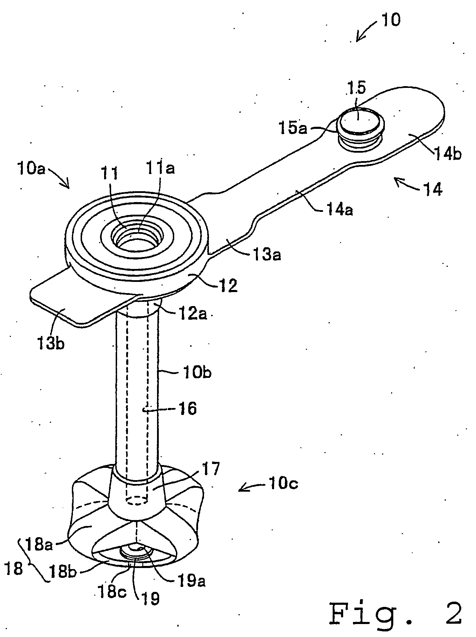

Hereinafter, referring to the drawings, an embodiment of the present invention will be described. Fig. 1 shows a state in which a gastrostomy tube extension device 20 according to the present invention is mounted to a gastrostomy tube 10. The gastrostomy tube 10 includes an outer fixing member 10a, a tube member 10b connected to the center of a lower end surface of the outer fixing member 10a, and an inner fixing member 10c connected to the lower end of the tube member 10b which are formed of polyurethane as shown in Fig. 2. In the following description, the side of the outer fixing member 10a is referred to as the upper side, and the side of the inner fixing member 10c is referred to as the lower side.

The outer fixing member 10a includes a main body 12 including a thick ring-shaped body provided with a vertically penetrating hole 11 at the center thereof; a cylindrical joint member 12a projecting downward from the center of a lower surface of the main body 12, outer holding strips 13a, 13b projecting respectively from both sides of the lower end portion on the outer peripheral surface of the main body 12, and a lid member 14 connected to a distal portion of one of the outer holding strip 13a. Formed on the upper end portion on an inner peripheral portion of the hole 11 formed on the main body 12 is an engaging groove 11a extending along the circumference thereof. Each of the outer holding strips 13a, 13b is formed into a thin plate of a substantially square shape in plan view extending horizontally from the outer peripheral surface of the main body 12 (the boundary between the outer holding strip 13a and the lid member 14 is a straight line).

The lid member 14 includes a band-shaped joint member 14a joined to the distal portion of the outer holding strip 13a and a plug member 15 provided at the distal end of the band-shaped joint member 14a. The band-shaped joint member 14a has flexibility and can bend so as to rotate in the vertical direction about the joint portion with respect to the outer holding strip 13a, able to bend at a steep angle. The distal portion of the band-shaped joint member 14a is formed with a wide portion 14b having wider width than the proximal side (at outer holding strip 13a), and the plug member 15 is provided on the wide portion 14b. The plug member 15 is provided on the wide portion 14b so as to face the hole 11 when the band-shaped joint member 14a is bent and the wide portion 14b is positioned on an upper surface of the main body 12.

The plug portion 15 is formed into a cylindrical shape which can be inserted into the hole 11 and is short in axial direction, and a projection 15a which can detachably engage the groove 11a of the hole 11 is provided along the circumference thereof. Therefore, the groove 11a and the projection 15a can be engaged with each other by bending the band-shaped joint member 14a and pressing the plug member 15 against the hole 11, and consequently, the hole 11 of the main body 12 can be clogged. Also, by pulling the wide portion 14b of the band-shaped joint member 14a forcedly upward from this state and releasing the engagement between the plug member 15 and the hole 11, the hole 11 of the main body 12 can be opened. The outer fixing member 10a configured as described above has a function to prevent the gastrostomy tube 10 from pulling into the stomach by being fixed to the patient's skin surface.

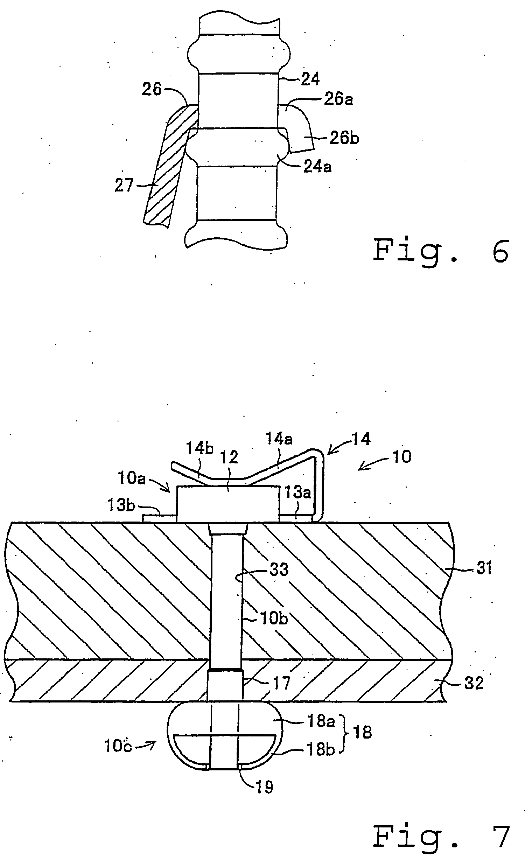

The upper end of the tube member 10b is fixed to the outer fixing member 10a while being inserted into the joint member 12a, and the interior of the tube member 10a defines a feed path 16 for feeding fluid substance such as liquid food. The upper end of the feed path 16 communicates with the hole 11 of the outer fixing member 10a, and the feed path 16 constitutes a fluid substance feed hole according to the present invention in cooperation with the hole 11 of the outer fixing member 10a. The tube member 10b is provided with elasticity, and hence is narrowed by being pulled, and returns to its original state when released from the pulled force. The tube member 10b has a function to prevent liquid or the like from leaking from the stomach by being positioned at a hole 33 (see Fig. 7) formed on the abdomen of the patient when it is placed in the hole 33.

The inner fixing member 10c includes a cylindrical joint member 17 connected to the lower end of the tube member 10b and an inner holding piece 18 connected to the lower end opening edge of the joint member 17. The joint member 17 is formed into a cylindrical shape which can cover the lower end portion of the outer peripheral surface of the tube member 10b, and is fixed to the tube member 10b in a state in which the lower end of the tube member 10b is inserted therein. The upper portion of the inner holding piece 18 is connected to the lower peripheral edge of the joint member 17 and includes a substantially dome-shaped contact portion 18a which comes into contact with the stomach wall when the inner fixing member 10c is positioned in the stomach. The lower portion of the inner holding piece 18 includes a plurality of band-shaped joint members 18b, in this embodiment four, joined to the lower edge of the contact portion 18c and a connecting portion 18c for connecting the distal portions of the respective joint members 18b.

Formed at the center portion of the upper surface of the contact portion 18a is a hole which communicates with the feed path 16 of the tube member 10b. The hole constitutes the fluid substance feed hole together with the hole 11 of the outer fixing member 10a and the feed path 16. The center portion of the upper surface of the contact portion 18a with which the stomach wall comes into contact is formed into a flat shape. The plurality of joint members 18b are provided at regular intervals around the circumference at the lower end of the contact portion 18a, extend separately axially from the lower edge of the contact portion 18a downward respectively, and then are converged at a lower position of the center axis of the tube member 10b to constitute the connecting portion 18c and thus are fixed together.

In other words, the connecting portion 18c connects the respective joint members 18b with each other by joining the lower end portions of the respective joint members 18b, and is positioned at a lower location of the center axis of the tube member 10b. Therefore, the inner holding piece 18 is formed into substantially a spherical shape as a whole with the pairs of opposing joint members 18b, 18b, and the outline of the contact portion 18a forming circles respectively.

The contact portion 18a and the respective joint members 18b are formed of soft resilient material having flexibility, and are maintained in a substantially spherical shape as a whole by the resiliency thereof as shown in Fig. 2 in a normal state. However, when the connecting portion 18c is pressed downward, it is expanded to a straight narrow state. In comparison with the sub-portions of the contact portion 18a above the respective joint members 18b, the sub-portions other than the above-described sub-portions are thinner.

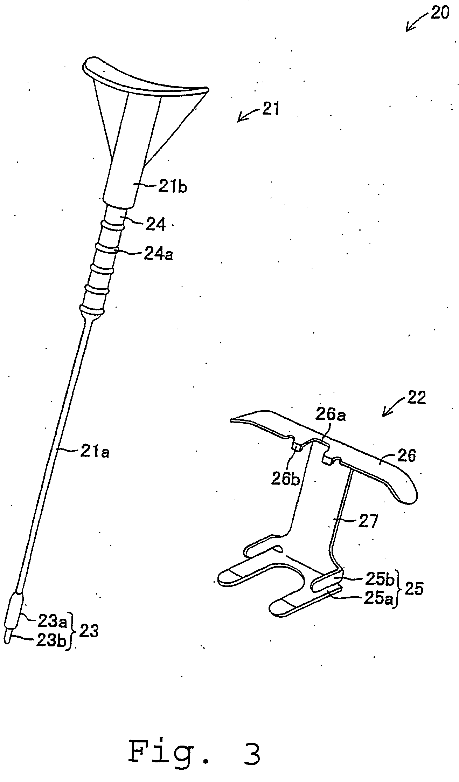

Therefore, the contact portion 18a is constructed of the portion extending from the joint members 18b and the thinned portions therebetween, and hence when the connecting portion 18c is pulled downward, it is folded to a specific shape and is easily narrowed. The connecting portion 18c is formed at the center with a hole, and an engaging portion 19 of a short cylindrical shape is fixed to this hole. The engaging portion 19 is formed at the center thereof with a hole 19a for positioning the distal portion of a rod (see Fig. 3) 21 described later. The inner fixing member 10c configured as described above has a function to be fixed to the inner surface of the stomach wall of the patient and prevents the gastrostomy tube 10 from leaving the patient's body.

The gastrostomy tube extension device 20 includes a rod 21 used as the pushing rod, and an engaging member 22 according to the present invention as shown in Fig. 3. The rod 21 is provided with a main body 21a formed of a rod-shaped member possibly formed of stainless steel, and a holding portion 21b possibly formed of plastic. The main body 21a is provided with a pushing portion 23, possibly formed of plastic, attached at the distal end thereof. The pushing portion 23 includes a secured portion 23a fixed to the main body 21a which covers the peripheral surface of the lower end portion of the main body 21a and a push-insertion piece 23b extending downward from the lower end of the secured portion 23a.

The outer diameter of the secured portion 23a is set to be larger than the diameter of the hole 19a of the engaging portion 19, and the diameter of the push-insertion piece 23b is set to be smaller than the diameter of the hole 19a. Therefore, when the rod 21 is inserted downward from the hole 11 of the gastrostomy tube 10, the push-insertion piece 23b is inserted into the hole 19a, and the secured portion 23a is located in such a manner that its lower surface contacts the upper surface of the engaging portion 19. Accordingly, when the rod 21 is pressed downward into the gastrostomy tube 10, the inner fixing member 10c extends and narrows.

A cylindrical portion 24 configured integrally with the holding portion 21b is formed on the upper portion of the main body 21a and below the holding portion 21b so as to cover the entire circumference of the main body 21a, and a plurality of engaging step portions 24a as the positioning engaging portions of the present invention are formed on the outer peripheral surface thereof. Each engaging step portion 24a is a ring-shaped projection possibly of substantially triangular shape in vertical cross-section formed on the peripheral surface of the cylindrical portion 24 all along the circumference thereof, and possibly five of these engaging step portions 24a may be formed at regular intervals along the axial direction of the cylindrical portion 24. The upper surface of the holding portion 21b is ideally formed into a curved surface depressed into an arcuate shape for allowing the operator's hand or finger, in particular, the thumb to fit for operation.

The engaging member 22 is formed into a shape as shown in Fig. 3 possibly by machining a stainless steel plate, and includes a lower engaging portion 25 as the first engaging portion of the present invention, and an upper engaging portion 26 as the second engaging portion of the present invention. The lower engaging portion 25 and the upper engaging portion 26 are connected by a joint strip 27 of a vertically elongated rectangular plate shape. The lower engaging portion 25 includes a holding strip 25a of substantially U-shape in plan view which is formed from the lower end of the joint strip 27, extending horizontally toward the near side of the drawing and orthogonally to the joint strip 27, and a pair of claw portions 25b provided in parallel with the holding strip 25a so as to extend from the both sides of the lower end portion of the joint strip 27, orthogonally to the joint strip 27 and at a distance from the holding strip 25a. The inside of the substantially U-shape of the holding strip 25a is formed into a recess which can accommodate the joint member 12a of the outer fixing member 10a and the distance between the holding strip 25a and the claw portion 25b is set to a distance which can clamp the outer holding strips 13a, 13b.

The upper engaging portion 26 is formed so as to extend from the upper end of the joint strip 27 horizontally toward the near side of the drawing and orthogonally to the joint strip 27, and is configured by a laterally elongated part extending from both sides of the joint strip 27. The length of the upper engaging portion 26 in the fore-and-aft direction is set to be short, relative to the length of the holding strip 25a, and is formed with an engaging recess 26a in the front center which can engage the respective engaging step portions 24a. A pair of projections 26b for preventing the engagement with the engaging step portion 24a from being released project downward at both sides of the engaging recess 26a of the front portion of the upper engaging portion 26. In addition, laterally both side portions of the upper engaging portion 26 may be curved downwardly so as to facilitate operation performed with the operator's hand hooked therewith, and the distal portion of the holding strip 25a may be curved upward so as to prevent engagement with the outer holding strips 13a, 13b from being released.

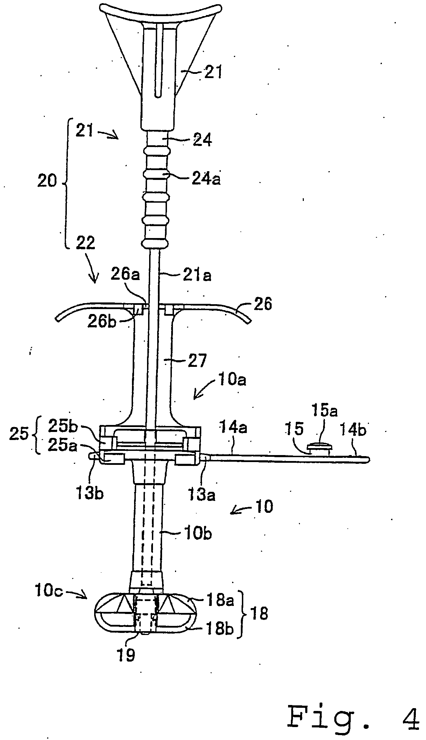

In this structure, in order to place the gastrostomy tube 10 in the hole 33 formed on the abdominal part of the patient, the wide portion 14b is pulled to open the hole 11 of the outer fixing member 10a and the rod 21 is inserted from the hole 11 toward the lower side of the tube member 10. Then, the push-insertion piece 23b of the rod 21 is aligned with the engaging portion 19 of the inner fixing member 10c and pushed into the hole 19a. Subsequently, the engaging member 22 is assembled to the gastrostomy tube 10 and the rod 21 in a state in which the outer holding strips 13a, 13b of the gastrostomy tube 10 in this state are clamped between the holding strip 25a and the claw portions 25b and the main body 21a is positioned in the engaging recess 26a to obtain the state shown in Fig. 1 or Fig. 4 (the direction of the gastrostomy tube 10 is different from Fig. 1).

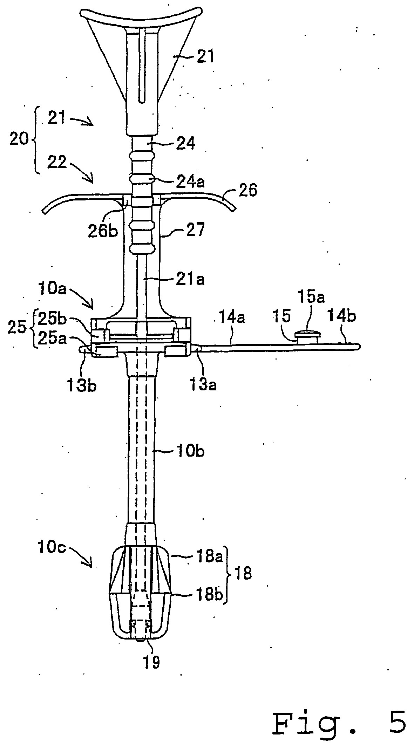

Subsequently, in a state in which the operator holds down the upper surface of the holding portion 21b by hand to prevent the push-insertion piece 23b from coming apart from the hole 19a, the operator hooks his fingers with the lower surface of the upper engaging portion 26 and pulls the engaging member 22 upward so that the edge of the engaging recess 26a is engaged with the predetermined engaging step portion 24a, for example, the engaging step portion 24a located at the center. Accordingly, as shown in Fig. 5, the inner fixing member 10c extends straight and is narrowed, and hence the tube member 10b and the inner fixing member 10c take a shape similar to one single rod.

In this case, not only the inner fixing member 10c, but also the tube member 10b is brought into an extended state. The gastrostomy tube 10 and the gastrostomy tube extension device 20 are assembled in a state in which the outer holding strips 13a, 13b are prevented from separating from the holding strip 25a by the curved portion at the distal end of the holding strip 25a, and the engaging step portion 24a of the rod 21 is prevented from separating from the engaging recess 26a by the projection 26b as shown in Fig. 6.

Subsequently, the gastrostomy tube 10 is inserted into the hole 33 formed on an abdominal wall 31 and a stomach wall 32 of the patient shown in Fig. 7 in the extended state as shown in Fig. 5. When the inner fixing member 10c enters the patient's stomach, the operator pulls the engaging member 22 upward while hooking his/her finger on the lower surface of the upper engaging portion 26, and separates the engaging recess 26a from the engaging step portion 24a as well as separating the holding strip 25a and the claw portion 25b from the outer holding strips 13a, 13b, whereby the engaging member 22 is removed from the gastrostomy tube 10. Then, the operator pulls out the rod 21 from the gastrostomy tube 10, and then bends the band-shaped joint member 14a to press the plug member 15 against the hole 11 to clog the hole 11 of the main body 12.

Accordingly, as shown in Fig. 7, the inner fixing member 10c returns to the original substantially spherical shape due to its resiliency, and the upper surface of the contact portion 18a comes into contact with the inner surface of the stomach wall 32. The tube member 10b is also restored to the original state. Consequently, the gastrostomy tube 10 is prevented from leaving the hole 33, and maintains the state of being attached to the abdomen of the patient. The portions of the abdominal wall 31 and the stomach wall 32 near the hole 33 are fixed so as not to be displaced in position from each other. In this case, it is preferable to leave a slight gap between the surface of the abdominal wall 31 and the lower surface of the outer fixing member 10a, so that a slight degree of freedom is provided between the gastrostomy tube 10 and the hole 33.

Then, when feeding fluid substance such as liquid food or nutrient preparation to the patient, the hole 11 of the outer fixing member 10a is opened, and a fluid feed tube (not shown) is connected to the hole 11. In this state, the fluid substance is fed from an opening at the end of the fluid feed tube into the fluid feeding tube. Consequently, the fluid substance is fed from the fluid feed tube via the hole 11 and the feed path 16 into the patient's stomach. In this case, the fluid substance coming out from the lower end opening of the tube member 10b passes from inside the inner fixing member 10c through the respective joint members 18b into the stomach. After use, the operator removes the fluid feed tube from the outer fixing member 10a and closes the hole 11.

When a physical property of the gastrostomy tube 10 is changed, such as expansion after after a certain period of use, and hence replacement is needed, the rod 21 and the engaging member 22 are mounted to the gastrostomy tube 10 in the abdomen of the patient as in the aforementioned operation. In this case, when the gastrostomy tube 10 has expanded, the engaging recess 26a of the engaging member 22 is engaged at the uppermost engaging step portion 24a or the second engaging step portion 24a from the top. Accordingly, the inner fixing member 10c can be brought into a narrow width which is suitable for its removal. The gastrostomy tube 10 is taken out from the patient's body together with the rod 21 and the engaging member 22, with the inner fixing member 10c narrowed as described above. Then, the new gastrostomy tube 10 is attached to the patient's body as in the aforementioned operation.

As described above, the gastrostomy tube extension device 20 according to this embodiment includes the rod 21 and the engaging member 22. Then, the rod 21 is provided at the lower end thereof with the push-insertion piece 23b which is engageable with the hole 19a of the engaging portion 19 provided on the gastrostomy tube 10, and on the peripheral surface of the upper portion of the rod 21 with the engaging step portions 24a. The engaging member 22 is provided with the lower engaging portion 25 engageable with the outer holding strips 13a, 13b of the gastrostomy tube 10 and the upper engaging portion 26 engageable with the engaging strips 24a of the rod 21.

Therefore, when the rod 21 is inserted into the gastrostomy tube 10, and the outer holding strips 13a, 13b are engaged with the lower engaging portion 25, by pulling the engaging member 22 upward and causing the upper engaging portion 26 to engage with the predetermined engaging step portion 24a, the inner fixing member 10c is maintained in a thinned state. Therefore, the operator can concentrate on the insertion or removal operation without paying attention to the degree of extension of the gastrostomy tube 10. Consequently, insertion and removal of the gastrostomy tube 10 into/from the hole 33 on the patient are made easy.

The distal portion of the holding strip 25a of the lower engaging portion 25 is bent upward and hence it is hard for the lower engaging portion 25 to separate from the outer holding strips 13a, 13b, and a pair of the projections 26b are formed at the ends of the opening of the engaging recess 26a on the front portion of the upper engaging portion 26, so that it is hard for the upper engaging portion 26 to separate from the engaging strips 24a of the rod 21. Therefore, the gastrostomy tube extension device 20 maintains stable extension of the gastrostomy tube 10, never separating from the gastrostomy tube 10.

Also, the engaging step portions 24a may be formed at regular intervals, and the length of the gastrostomy tube 10 can be extended by stages. Accordingly, versatility is provided, and hence one type of gastrostomy tube extension device 20 may be sufficient for insertion and removal of the various gastrostomy tubes. Also, since the engaging step portion 24a to be engaged with the engaging recess 26a may be selected according to the change in the expansion or other physical properties of the gastrostomy tube 10, the operation in the optimal state is achieved.

The gastrostomy tube extension device according to the present invention is not limited to the aforementioned embodiment, and may be changed as needed within the technical scope of the present invention. For example, although the gastrostomy tube 10 and the engaging member 22 in the aforementioned embodiment are separate members, they may be formed integrally. In the aforementioned embodiment, although five engaging step portions 24a are formed on the rod 21, the number of the engaging step portions 24a may be plural of four or smaller, or six or larger, or may be single. The joint members 18c may also vary in number or shape, even perhaps being formed into one member, within the scope of this disclosure.

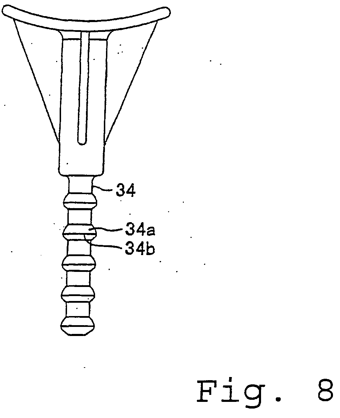

In addition, it is also possible to form the engaging step portion 24a to have an angular portion having a ridge line 34b at the center like the engaging step portion 34a formed into the cylindrical portion 34 shown in Fig. 8 instead of a smoothly curved shape. This arrangement further ensures engagement with respect to the engaging recess 26a. Further, instead of a ring shape, the engaging step portion may be formed of a projection projecting from a predetermined position on the cylindrical portion or may be formed of a recess with which the upper engaging portion 26 can engage. Also, the shapes or materials of other portions constituting the gastrostomy tube 10 and the gastrostomy tube extension device 20 may also be changed as needed.

### [Brief Description of the Drawings]

[Fig. 1] Fig. 1 is a perspective view showing a state in which a gastrostomy tube extension device according to a first embodiment of the present invention is mounted to a gastrostomy tube.
[Fig. 2] Fig. 2 is a perspective view of the gastrostomy tube.
[Fig. 3] Fig. 3 is a perspective view of the gastrostomy tube extension device.
[Fig. 4] Fig. 4 is a front view showing a state in which the gastrostomy tube extension device is mounted to the gastrostomy tube.
[Fig. 5] Fig. 5 is a front view showing a state in which the gastrostomy tube is extended by the gastrostomy tube extension device.
[Fig. 6] Fig. 6 is a cross-sectional view showing a state in which an engaging step portion and an engaging recess are engaged.
[Fig. 7] Fig. 7 is a cross-sectional view showing a state in which the gastrostomy tube is placed in the patient's body.
[Fig. 8] Fig. 8 is a front view showing a modification of the engaging step portion.

## Claims

1. A gastrostomy tube extension device (20) used for inserting or taking out a gastrostomy tube (10) into/from a hole (33) on a patient formed between the skin surface and the inner surface of a stomach wall; the gastrostomy tube (10) comprising:
an outer fixing member (10a) to be installed at the hole of the patient on the skin surface side,
an inner fixing member (10c) to be installed on the inner side of the stomach wall which is formed of resilient material which becomes elongated and narrowed when the center of the distal end thereof is pushed toward the distal end; and
a tube member (10b) for connecting the outer fixing member (10a) and the proximal portion of the inner fixing member (10b), and a fluid substance feed hole (16) extending from the outer fixing portion (10a) to the inner fixing portion (10b),
said extension device (20) comprising:
a pushing rod (21) comprising a rod member (21 a) which can be inserted into the fluid substance feed hole, is capable of pushing the center portion of the distal end of the inner fixing member (10b) toward the distal end with the distal end portion (23) thereof, and is formed with a positioning engaging portion (24a) on the peripheral surface of the proximal portion; and
an engaging member (22) including: a first engaging portion (25) which is capable of engaging the outer fixing member (10a), and a second engaging portion (26) which is capable of engaging the positioning engaging portion (24a) of the pushing rod (21), the engaging member (22) being arranged to elongate and narrow the inner fixing portion (10b) when the pushing rod (21) is inserted into the fluid substance feed hole and the outer fixing member (10a) and the first engaging portion (25), and the positioning engaging portion (24a) and the second engaging portion (26) are respectively engaged with each other, **characterized in that**
a plurality of the positioning engaging portions are provided along the axial direction of the pushing rod (21) and wherein each of the respective plurality of positioning engaging portions comprise an engaging step portion that is a ring-shaped projection.

2. The gastrostomy tube extension device according to claim 1 wherein the engaging member (22) includes a joint member being connected to the gastrostomy tube.

3. The gastrostomy tube extension device according to any of the preceding claims further comprising a gastrostomy tube (10), the gastrostomy tube (10) comprising: an outer fixing member (10a) to be installed at the hole of the patient on the skin surface side, an inner fixing member (10c) to be installed on the inner side of the stomach wall which is formed of resilient material which is elongated and narrowed when the center of the distal end thereof is pushed toward the distal end; and a tube member (10b) for connecting the outer fixing member (10a) and the proximal portion of the inner fixing member (10b), and a fluid substance.

4. The gastrostomy tube extension device according to any of the preceding claims wherein the plurality of positioning engaging portions (24a) are formed at regular intervals along the pushing rod (21).

## Patentansprüche

1. Gastrostomieschlauchverlängerungsvorrichtung (20), die zum Einführen oder Herausnehmen eines Gastrostomieschlauchs (10) in eine/aus einer Öffnung (33) an einem Patienten verwendet wird, die zwischen der Hautoberfläche und der Innenfläche der Magenwand gebildet ist; wobei der Gastrostomieschlauch (10) Folgendes umfasst:
ein äußeres Befestigungselement (10a) zum Installieren an der Öffnung des Patienten auf der Hautoberflächenseite,
ein inneres Befestigungselement (10c) zum Installieren an der Innenseite der Magenwand, das aus elastischem Material gebildet ist, das sich streckt und schmaler wird, wenn die Mitte seines distalen Endes zum distalen Ende geschoben wird; und
ein Schlauchelement (10b) zum Verbinden des äußeren Befestigungselements (10a) und des proximalen Abschnitts des inneren Befestigungselements (10b), und eine Fluidsubstanzeinleitöffnung (16), die sich von dem äußeren Befestigungsabschnitt (10a) zum inneren Befestigungsabschnitt (10b) erstreckt,
wobei die Verlängerungsvorrichtung (20) Folgendes umfasst:
eine Schubstange (21), die ein Stangenelement (21a) umfasst, das in die Fluidsubstanzeinleitöffnung eingeführt werden kann, den mittleren Abschnitt des distalen Endes des inneren Befestigungselements (10b) mit seinem distalen Endabschnitt (23) zum distalen Ende schieben kann und mit einem Positionierungseingriffabschnitt (24a) an der Umfangsfläche des proximalen Abschnitts gebildet ist; und
ein Eingriffelement (22), beinhaltend: einen ersten Eingriffabschnitt (25), der in Eingriff mit dem äußeren Befestigungselement (10a) treten kann, und einen zweiten Eingriffabschnitt (26), der in Eingriff mit dem Positionierungseingriffabschnitt (24a) der Schubstange (21) treten kann, wobei das Eingriffelement (22) dazu angeordnet ist, den inneren Befestigungsabschnitt (10b) zu verlängern und schmaler zu machen, wenn die Schubstange (21) in die Fluidsubstanzeinleitöffnung und das äußere Befestigungselement (10a) und den ersten Eingriffabschnitt (25) eingeführt wird und der Positionierungseingriffabschnitt (24a) und der zweite Eingriffabschnitt (26) jeweils miteinander in Eingriff gelangen, **dadurch gekennzeichnet, dass**
eine Mehrzahl von Positionierungseingriffabschnitten entlang der Axialrichtung der Schubstange (21) vorgesehen ist und wobei jeder der jeweiligen Mehrzahl von Positionierungseingriffabschnitten einen Eingriffstufenabschnitt umfasst, der ein ringförmiger Vorsprung ist.

2. Gastrostomieschlauchverlängerungsvorrichtung nach Anspruch 1, wobei das Eingriffelement (22) ein Verbindungselement beinhaltet, das mit dem Gastrostomieschlauch verbunden ist.

3. Gastrostomieschlauchverlängerungsvorrichtung nach einem der vorangehenden Ansprüche, ferner umfassend einen Gastrostomieschlauch (10), wobei der Gastrostomieschlauch (10) Folgendes umfasst: ein äußeres Befestigungselement (10a) zum Installieren an der Öffnung des Patienten an der Hautoberflächenseite, ein inneres Befestigungselement (10c) zum Installieren an der Innenseite der Magenwand, das aus elastischem Material gebildet ist, das verlängert und schmaler gemacht wird, wenn die Mitte seines distalen Endes zum distalen Ende geschoben wird; und ein Schlauchelement (10b) zum Verbinden des äußeren Befestigungselements (10a) und des proximalen Abschnitts des inneren Befestigungselements (10b), und eine Fluidsubstanz.

4. Gastrostomieschlauchverlängerungsvorrichtung nach einem der vorangehenden Ansprüche, wobei die Mehrzahl von Positionierungseingriffabschnitten (24a) in regelmäßigen Intervallen an der Schubstange (21) gebildet ist.

## Revendications

1. Dispositif d'extension de sonde de gastrostomie (20) utilisé pour insérer ou retirer une sonde de gastrostomie (10) dans/à partir d'un orifice (33) sur un patient, formé entre la surface de la peau et la surface interne d'une paroi d'estomac;
la sonde de gastrostomie (10) comprenant:
un élément de fixation externe (10a) destiné à être installé à l'orifice du patient du côté surface de la peau,
un élément de fixation interne (10c) destiné à être installé sur le côté interne de la paroi d'estomac, qui est formé d'un matériau élastique qui devient allongé et rétréci, lorsque le centre de l'extrémité distale de celui-ci est poussé vers l'extrémité distale; et
un élément de sonde (10b) pour connecter l'élément de fixation externe (10a) et la partie proximale de l'élément de fixation interne (10b), et un orifice d'alimentation en substance liquide (16) s'étendant de la partie de fixation externe (10a) jusqu'à la partie de fixation interne (10b),
ledit dispositif d'extension (20), comprenant:
une tige de poussée (21) comprenant un élément de tige (21a) qui peut être inséré dans l'orifice d'alimentation en substance liquide est capable de pousser la partie centrale de l'extrémité distale de l'élément de fixation interne (10b) vers l'extrémité distale avec la partie d'extrémité distale (23) de celui-ci, et est formée d'une partie d'engagement de positionnement (24a) sur la surface périphérique de la partie proximale ; et
un élément d'engagement (22) comprenant : une première partie d'engagement (25) qui est capable d'entrer en prise avec l'élément de fixation externe (10a), et une seconde partie d'engagement (26) qui est capable d'entrer en prise avec la partie d'engagement de positionnement (24a) de la tige de poussée (21), l'élément d'engagement (22) étant agencé de manière à allonger et rétrécir la partie de fixation interne (10b) lorsque la tige de poussée (21) est insérée dans l'orifice d'alimentation en substance liquide et l'élément de fixation externe (10a) et la première partie d'engagement (25), et la partie d'engagement de positionnement (24a) et la seconde partie d'engagement (26) entrent respectivement en prise l'une avec l'autre, **caractérisé en ce que**
une pluralité de parties d'engagement de positionnement est prévue le long de la direction axiale de la tige de poussée (21) et dans lequel chacune de la pluralité respective des parties d'engagement de positionnement comprend une partie d'étape d'engagement qui est une projection en forme d'anneau.

2. Dispositif d'extension de sonde de gastrostomie selon la revendication 1 dans lequel l'élément d'engagement (22) comprend un élément de joint qui est connecté à la sonde de gastrostomie.

3. Dispositif d'extension de sonde de gastrostomie selon une quelconque des revendications précédentes, comprenant en outre une sonde de gastrostomie (10), la sonde de gastrostomie (10) comprenant: un élément de fixation externe (10a) destiné à être installé au niveau de l'orifice du patient sur le côté surface de la peau, un élément de fixation interne (10c) destiné à être installé sur le côté interne de la paroi d'estomac, qui est formé d'un matériau élastique qui est allongé et rétréci lorsque le centre de l'extrémité distale de celui-ci est poussé vers l'extrémité distale; et un élément de sonde (10b) pour connecter l'élément de fixation externe (10a) et la partie proximale de l'élément de fixation interne (10b), et une substance liquide.

4. Dispositif d'extension de sonde de gastrostomie selon l'une quelconque des revendications précédentes dans lequel la pluralité de parties d'engagement de positionnement (24a) est formée à intervalles réguliers le long de la tige de poussée (21).
